# EUROPEAN PATENT SPECIFICATION

(11) **EP 1 765 843 B1**
(45) Date of publication and mention of the grant of the patent: **18.05.2016**
(21) Application number: 05756858.6
(22) Date of filing: 29.06.2005
(51) Int. Cl.: C07H 15/18, C08K 5/06, C08K 7/00

(54) **PROCESS FOR PREPARING A PULVERULENT ALDITOL ACETAL COMPOSITION**
VERFAHREN ZUR HERSTELLUNG EINER PULVERFÖRMIGEN ALDITOLACETALZUSAMMENSETZUNG
PROCEDE DESTINE A PREPARER UNE COMPOSITION D'ACETAL D'ALDITOL PULVERULENTE

(30) Priority: 09.07.2004 FR 0407714
(43) Date of publication of application: 28.03.2007
(73) Proprietor: BASF SE, 67056 Ludwigshafen am Rhein (DE)
(72) Inventor: WYART, Hervé, F-62149 Cuinchy (FR); STOLL, Klaus, 79589 Binzen (DE)
(74) Representative: BASF IP Association
(86) International application number: PCT/EP2005/053052
(87) International publication number: WO 2006/005681

(56) References cited:
- EP-A- 0 361 087
- EP-A- 0 569 198
- EP-A- 0 651 006
- EP-A- 0 962 459
- WO-A-99/33776
- PATENT ABSTRACTS OF JAPAN vol. 015, no. 181 (C-0830), 9 May 1991 (1991-05-09) & JP 03 043466 A (UBE IND LTD), 25 February 1991 (1991-02-25)
- PATENT ABSTRACTS OF JAPAN vol. 008, no. 106 (C-223), 18 May 1984 (1984-05-18) & JP 59 020363 A (ASAHI KASEI KOGYO KK), 2 February 1984 (1984-02-02)
- PATENT ABSTRACTS OF JAPAN vol. 008, no. 106 (C-223), 18 May 1984 (1984-05-18) & JP 59 020362 A (ASAHI KASEI KOGYO KK), 2 February 1984 (1984-02-02)

## Description

The present invention relates to a novel process for preparing a powdery or pulverulent alditol acetal composition.

It also relates to a pulverulent alditol acetal composition that can be obtained according to this process and that has improved characteristics, in particular density, particle size and/or flow characteristics.

It is widely known that alditol acetals such as dibenzylidene sorbitol and its derivatives can be used as additives, and in particular as nucleating agents or clarifying agents, for plastics such as polyolefins.

These products are also used as gelling agents or viscosity-modifying agents for various materials such as compositions for cosmetic or pharmaceutical use, adhesive compositions or paints. These compositions can also be formulated in articles such as sticks of adhesive or of cosmetic material, for example of deodorants or air fresheners.

One of the main drawbacks associated with the industrial use of alditol acetals concerns the very pulverulent and very adhesive nature of these products.

This nature, that in particular produces flow problems, is linked in particular to the fact that these products, at the time of their synthesis, crystallize naturally in the form of individual or "primary" particles of very fine size and of more or less marked hydrophobic nature.

By way of example, sorbitol diacetals crystallize naturally in the form of needles or rods less than 15 µm, generally between 5 and 10 µm, in length and less than 2 µm, generally between 0.3 and 1 µm, in diameter.

This fine particle size is generally conserved during the subsequent industrial steps of neutralization, washing and then filtration of the synthesis medium.

It is also conserved during all or part of the subsequent step of industrial drying depending, inter alia, on the drying conditions and on the drying device used, and in particular on the possible need to remove any trace of organic solvent that may be used during the synthesis of the aldetol diacetal.

At a given moment, in fact, there may or may not be formation of agglomerates of primary particles, within the powder during drying.

In any event, whether or not there is formation of such agglomerates during said drying, the industrial alditol acetal powders placed on the market all exhibit this very pulverulent and adhesive nature. This results in difficulties in the industrial operations of transport, bagging, metering, mixing, storage, cleaning and the like.

In particular, alditol acetal powders readily form "arches", i.e. prone to "bridging" within storage tanks and/or transport pipes. This prevents or impedes the conveying, metering and/or evacuation thereof at the feed hoppers and the metering systems of industrial units, for example of units intended for the preparation of plastics or of additives for plastics.

The term "plastics" is intended especially to mean polyolefins, in particular all polymers based on propylene and/or on ethylene, polyamides, thermoplastic polyesters, vinyl resins, acrylic resins and mixtures thereof.

This problem of flow of the pulverulent forms of alditol acetals could up until now only be solved, in the industrial practice, by making use, at the manufacturers and/or users of these products, of adapted devices, such as specific pneumatic feeding systems or hoppers whose inside coating is covered with TEFLON.

Besides the fact that such devices can be expensive and/or complex, they do not always make it possible to prevent the above mentioned "arching" phenomena or, at the very least, clogging phenomena in hoppers, metering systems or, downstream, spiral conveyor screws conveying the mixtures to the units for preparing the plastics or the gelled materials.

Moreover, actually within the compositions of materials into which it has been possible to introduce and meter them satisfactorily, alditol acetals can show a poor ability to disperse. This phenomenon can induce, in particular in plastics and gelled materials, a more or less pronounced impairment or heterogeneity of the final characteristics of said materials (general appearance, organoleptic, optical, mechanical, etc., characteristics).

Various technologies have been proposed in order to overcome the above mentioned problems of poor dispersibility of alditol acetals, in particular of dibenzylidene sorbitol and of its derivatives. JP-A-60/101,131 has, for example, described the drying and then the fine milling of an alditol diacetal pretreated with terephthalic acid in the presence of an anionic surfactant.

Lyophilization of an alditol diacetal gel in a solvent has also been recommended, as disclosed in JP-A-62/253,646. The lyophilized product thus obtained has a "packed bulk" density that is low, namely of the order of 100 g/l, in any case very significantly decreased (by a factor of approximately 3) compared with the starting alditol diacetal.

More recently, EP-A-569,198 has proposed the ultrafine milling of alditol diacetal compositions containing agglomerates on suitable devices, combining a fluidized bed and a very high speed turbine (jet mill), with a view to obtaining products having an "ultrafine" particle size, in particular characterized by a "d 97" index at most equal to 30 µm.

On reading Example 2 of that document, it appears that such an ultrafine milling treatment applied to dibenzylidene sorbitol of the trade mark "Millad® 3905" is accompanied by a very significant decrease (by a factor of approximately 3.3) in the "packed bulk" density of the product.

Even more recently, other forms of technology have been recommended with a view to improving the dispersibility of alditol diacetals, and in particular:
the preparation, by drying/spraying, of a fine powder based on a mixture of an alditol diacetal and a particular phosphite, said mixture having been solubilized beforehand in a solvent (EP-A-651,006), or
the extensive drying and milling, in particular in an instantaneous drying device, of a wet alditol diacetal, the dried/milled product obtained having a very low (0.01%) residual water content and an apparent density of the order of approximately 200 g/l (JP-A-09/048,783).

It results therefrom that the means recommended for guaranteeing good dispersibility of alditol acetals in plastics and other materials are generally complex and/or expensive, or even dangerous, in particular due to the fact that they involve the use of specific drying materials, specific milling materials and/or specific solvents.

In addition, as indicated, these means generally have the aim or the effect of significantly decreasing the density of the alditol acetals, which increases the dusty nature and the dangerousness thereof (risks of explosion and inhalation) and deteriorates the ability thereof to flow.

Recently, various propositions have been made with a view to improving the flow properties of alditol diacetal compositions, without decreasing, or even while simultaneously improving, other physical, organoleptic and/or applicative characteristics of said compositions.

This is the case of WO-A-99/033,776, which recommends the use of an additive chosen from tocopherols and polyols with a view to improving the flow and/or the stability of such compositions.

Very preferably, the mixing of the additive and the alditol acetal powder is carried out under cold conditions and these cold conditions are maintained during the subsequent formulating and/or densification step, this step advantageously consisting of a compacting step that generates a very high densification of the product.

For its part, EP-A-962,459 describes alditol diacetal compositions in which a binder has been uniformly distributed, not only at the surface but inside said particles. Such a distribution can only be obtained after having carried out the process with prior swelling of the alditol diacetal in a solvent.

It is said that the solvent can be water, but, in practice, all the very many examples of that document envisage starting from an alditol diacetal powder that is subsequently, directly or indirectly, swollen in an organic solvent which may be polar (ethanol, methanol) and/or of the "alicyclic hydrocarbon" type (cyclohexane).

The dispersion or "slurry" of the alditol diacetal in the solvent is then mixed uniformly with the binder, the latter being, in most of said examples, a fatty acid or non-fatty acid.

This process is complicated since, before carrying out the granulation of the mixture, generally performed at a drying temperature at most equal to 80°C, it is advisable to eliminate any organic solvent that may be contained in said mixture.

The characteristics, in particular density, flow and particle size characteristics, of the granulated compositions obtained directly at the end of the drying step are not in any way given in detail since, according to these examples, said compositions are directly pulverized on a household mixer for 10 minutes with a view to re-obtaining powders. Only the characteristics of these final compositions thus pulverized are indicated.

EP-A-964,029 describes a process for granulating and formulating an alditol diacetal powder using a binder, under conditions, in particular pressure and temperature conditions, that should allow (very) high densification of the initial powder and aggregation of its constituent particles by means of the binder, the latter necessarily having to adhere to, or even to cover, completely or partly, each particle of diacetal.

The granulation/formulation under pressure of the alditol diacetal composition can in particular be carried out on an extruder as described in the examples of that patent.

In all these examples, use is made of alditol diacetal powders which, before extrusion, are mixed at relatively high temperature (180°C) with various binders. Details of the characteristics, in particular density, flow and particle size characteristics, of the intermediate mixture not yet granulated on the extruder are not given.

JP59-20362 also discloses compostions comprising dibenzylidenesorbitol mixed with an antioxidant.

A means has now been found, which makes it possible, simply, non-dangerously and relatively inexpensively, to provide alditol acetal compositions, in particular compositions of dibenzylidene sorbitol ("DBS") and of its derivatives, exhibiting physical, but also applicative characteristics corresponding to current industrial requirements, which means, in particular:
a) in no way requires the use and therefore the elimination of any organic solvent,
b) in no way requires the prior provision of an alditol acetal in the form of a powder, including of a fine powder,
c) in no way requires the subsequent provision of particular physical treatment means, such as devices for milling or pulverization, for extrusion, for compacting, for drying at very high temperatures (≥ 180°C), etc.,
d) makes it possible to obtain compositions whose density and particle size characteristics and whose non-adhesive nature are particularly suitable for industrial processes of transport, bagging, metering, mixing, storage, cleaning or the like,
e) makes it possible to obtain compositions whose applicative characteristics, for example in the fields of additives for plastics or gelled materials, are in no way impaired, or are even improved, and
f) makes it possible to obtain compositions whose composition and organoleptic characteristics are, from a regulatory but also industrial and commercial point of view, also entirely suitable for users' requirements.

This means is bringing together with one another two particular products namely 1) an alditol acetal composition selected both by virtue of its aqueous nature and by virtue of its concentration of solid materials or solids content (SC) and 2) one or more antioxidants.

More precisely, a subject of the present invention is a process for preparing a pulverulent alditol acetal composition, which comprises
a) mixing, in the presence or absence of other constituents,
   an aqueous alditol acetal composition having a solids content (SC) of between 40 and 75%, in particular of between 45 and 72%, relative to the weight of the total aqueous alditol acetal composition, and
   an antioxidant in solid or liquid form or an antioxidant composition in solid or liquid form, containing one or more antioxidants, and
b) subsequent drying of the mixture obtained under a).

For the purposes of the present invention, the term "alditol acetal" is intended to mean, in particular, alditol diacetals, especially those resulting from the dehydrocondensation of an alditol containing five or six carbon atoms with a benzoic aldehyde.

Said alditol can in particular be chosen from the group comprising sorbitol, xylitol, mannitol, ribitol, arabitol and iditol. It can be modified e.g. on the last carbon atom of its chain, for example by introduction of a carboxylic group, and can thus consist of a gluconate or a xylonate.

The benzoic aldehyde used for the purpose of preparing the alditol acetals that can be used according to the invention can in particular be benzaldehyde or 1-naphthaldehyde, or any one of their respective derivatives.

Preferably, the benzoic aldehyde used is a benzaldehyde or any one of its derivatives, for example those substituted, in one or more positions of the aromatic ring, with an alkyl, alkoxyl, bis-oxy-alkylene, hydroxyl, halogen, thioalkyl or sulphoalkyl group.

When the benzaldehyde is substituted in several places, its substituents may or may not be identical. The substituents may also form a carbo- or heterocyclic ring with the aromatic benzaldehyde moiety.

Advantageously, the benzaldehyde is substituted in one, two or three places with an alkyl, in particular methyl or ethyl, group, with a halogen group, in particular a chlorinated or fluorinated group, with a hydroxyl group and/or with an alkoxyl group, in particular a methoxy group. The substitution is in particular preferred at the 2- (ortho) position, the 3- (meta) position and/or the 4- (para) position of the benzaldehyde.

Preferably, the alditol acetal is an alditol diacetal chosen from the group comprising 1,3:2,4-di(benzylidene) sorbitol (hereinafter referred to as "DBS"), 1,3:2,4-di(benzylidene)xylitol (hereinafter referred to as "DBX") and derivatives thereof, in particular alkylated and/or halogenated derivatives, preferably chosen from DBS and its alkylated derivatives.

Particularly advantageous, the alditol diacetal is chosen from methylated derivatives of DBS, in particular those obtained by dehydrocondensation of sorbitol with a benzaldehyde that is methylated, at the very least, at the 3-position or 4-position of its ring (respective "meta" or "para" positions).

The alkyl substituted derivatives of DBS are preferably:
1,3:2,4-di-(4-ethylbenzylidene) sorbitol,
1,3:2,4-di-(4-methylbenzylidene) sorbitol,
1,3:2,4-di-(3-methylbenzylidene) sorbitol, and
1,3:2,4-di-(3,4-dimethylbenzylidene) sorbitol.

The expression "aqueous alditol acetal composition" is intended to mean any composition whose liquid phase contains no organic solvent or contains sufficiently little organic solvent(s). That means for example that the weight ratio of, firstly, water to all the organic solvents possibly present in said liquid phase is greater than 2/1, preferably greater than 3/1, and most particularly greater than 5/1 or 10/1. The aqueous alditol acetal composition is preferably free or essentially free of any organic solvent.

Characteristically, the solids content (SC) of said aqueous composition is between 40 and 75%, in particular between 45 and 72%, relative to the total weight of the aqueous composition. This SC can advantageously be between 45 and 65%. Further examples of SC are between 47 and 72%, between 48 and 72% and between 50 and 65%.

These values are preferably, in general, very significantly higher than those of the alditol acetal dispersions or "slurries" constituted by the synthesis media, in the aqueous phase, of these products.

In any case, these values differ fundamentally from the much higher (> 95%) SC values of the commercial alditol acetal powders.

The aqueous alditol acetal composition used in accordance with the invention is very advantageously provided as a wet material that is not formulated and not pulverulent and that does not flow freely.

According to a preferred embodiment, said composition is a filter cake obtained after synthesis, in an aqueous medium, of the alditol acetal.

The term "filter cake" is intended to mean any composition, that may or may not be neutralized, having undergone at least one step consisting of filtration, of pressing and/or of any other equivalent means of partial elimination of the aqueous phase contained in the dispersion or "slurry" constituted by the original synthesis medium, that may or may not be neutralized.

This elimination of aqueous phase can advantageously be carried out on a vacuum belt filter that may be equipped, in its end portion, with a belt press, also under vacuum.

In the course of being constituted from the original aqueous dispersion, said filter cake may or may not have undergone at least one washing step and/or at least one neutralization step, in particular by means of water and/or other aqueous compositions sprayed onto said cake. Very advantageously, the water used for these washing and neutralization processes is hot and has a temperature of between approximately 30 and 90°C, in particular of between approximately 40 and 85°C.

The filter cake used in accordance with the invention may or may not also have undergone at least one step consisting of fragmentation that is more or less coarse, but that does not however modify the wet and non-pulverulent nature thereof. Such a step may in particular be carried out by means of steel wires on which the cake fragments as it is unloaded from the belt of the filter or of the press. It may also be carried out on a device of the "clod-breaking" or "arch-breaking" type.

By way of example, said filter cake can consist of a composition of 1,3:2,4-di-(4-methylbenzylidene) sorbitol ("MDBS") having an SC of approximately 50% and a temperature of 45-50°C, resulting from filtration, washing (under hot conditions), neutralization (under hot conditions), pressing and, finally, coarse fragmentation steps, which steps are carried out using an aqueous suspension of MDBS with a relatively low SC (< 10%).

The filter cake can be prepared, for example, as described in US-A-5,023,354. Thus, a preferred embodiment of the present invention relates to a process wherein the aqueous alditol acetal composition used is prepared by a process comprising the following steps:
acetalizing in an aqueous medium comprising water as single solvent and in the presence of an acid catalyst by introducing in said medium under stirring a benzoic aldehyde and an alditol having 5 or 6 carbon atoms, the initial molar ratio of the benzoic aldehyde to the alditol being lower than 2/1,
optionally neutralizing by means of a base the resulting mixture which is in the form of an aqueous suspension consisting of a solid and of a liquid phase,
separating the solid phase and
optionally washing the separated solid phase with warm water, and
subsequently filtering to obtain a filter cake of the final product in its wet form,
the acid catalyst is selected from the group consisting of arylsulfonic acids,
the arylsulfonic acid and the benzoic aldehyde are in a molar ratio which is initially higher than 0.6, and
the acetalization is carried out at a temperature lower than about 45°C.

The following preferred embodiments relate to the preparation of the filter cake:
(1) the alditol is preferably introduced into the reaction medium in the form of an aqueous solution, the concentration in alditol of the latter not exceeding about 30%.
(2) The alditol, when being introduced into the aqueous medium, is in aqueous solution, the concentration in alditol of the said aqueous solution being between 20 and 30%.
(3) The acid catalyst is selected from the group consisting of phenylsulfonic and naphthylsulfonic acids.
(4) The acid catalyst is selected from the group consisting of paratoluenesulfonic acid, benzenesulfonic acid, 5-sulfosalicylic acid and naphthalenesulfonic acid.
(5) The benzoic aldehyde is a benzaldehyde unsubstituted or substituted on the phenyl nucleus by at least one substituent selected from the group consisting of lower alkyl groups having 1 to 4 carbon atoms.
(6) The benzoic aldehyde is benzaldehyde substituted on the phenyl nucleus by the methyl or the ethyl group.
(7) For the preparation of dibenzylidenesorbitol, the alditol is sorbitol and the benzoic aldehyde is benzaldehyde.
(8) For the preparation of bis[para-ethylbenzylidene]sorbitol, the alditol is sorbitol and the benzoic aldehyde is para-ethylbenzaldehyde.
(9) For the preparation of bis[para-methylbenzylidene]sorbitol, the alditol is sorbitol and the benzoic aldehyde is para-methylbenzaldehyde.
(10) For the preparation of bis[3,4-dimethylbenzylidene]sorbitol, the alditol is sorbitol and the benzoic aldehyde is 3,4-dimethylbenzaldehyde.
(11) For the preparation of dibenzylidenexylitol, the alditol is xylitol and the benzoic aldehyde is benzaldehyde.
(12) The initial molar ratio arylsulfonic acid/benzoic aldehyde is between 0.6/1 and 1.5/1.
(13) The initial molar ratio arylsulfonic acid/benzoic aldehyde is between 0.6/1 and 1/1.
(14) The acetalization is carried out at a temperature between 15° and 45°C.
(15) The acetalization is carried out at a temperature between 20° and 40°C.
(16) The acetalization is carried out at a temperature between 30° and 40°C.
(17) Once the acetalization has been conducted to its end, the resulting reaction medium is neutralized to a pH value between about 7 and 7.5 by means of an alkaline agent selected from the group consisting of sodium hydroxide, potassium hydroxide and sodium bicarbonate.

A preferred example for the preparation of the filter cake is described below:
Common conditions:
   Concentration of sorbitol: about 25% by weight.
   Catalyst: paratoluenesulfonic acid (APTS) molar ratio acid/sorbitol=1.25.
   Temperature: about 30°C.
   Time: about 5h30.

In a cylindrical 2 I reaction vessel equipped with a double casing and a rotating stirring device fitted with a 3-bladed turbine, there are introduced 728 g of an aqueous solution of sorbitol with 25% dry matter (1 mole), 215 g of paratoluenesulfonic acid (1.25 mole) and 190.8 g of benzaldehyde (1.8 mole).

This aqueous mixture is brought, under stirring, to a temperature of 30°C, then maintained under these conditions for a duration of 5 hours and 30 minutes approximately. The reaction medium thus obtained is neutralized by a solution of 10% sodium hydroxide until a pH-value in the vicinity of 7.2 is reached, then filtered under vacuum on a filter of the BUCHNER-type. The resulting filtration cake is then optionally resuspended in warm water (about 60°C), then filtered again. The filter cake obtained contains approximately 50% dry matter.

In accordance with the invention, the aqueous alditol acetal composition, for example the filter cake described above, is subsequently mixed with an antioxidant in solid or liquid form or an antioxidant composition in solid or liquid form.

The term antioxidant is intended to mean in particular all antioxidants already described or used for stabilizing plastics or gelled materials, including in combination with alditol acetals.

These compounds may in particular correspond to those described in Chapter 1 entitled "Antioxidants" of the 5th Edition of the book "Plastics Additives Handbook" (2001) Carl Hanser Verlag, Munich (Germany), or to any mixture of at least any two of these compounds.

They may in particular be compounds whose chemical structure is in accordance with one of those given in the passage corresponding to pages 98 to 108 of Chapter 1 of the abovementioned book.

According to a first variant, the antioxidant is a phenolic antioxidant, preferably not containing a long alkyl chain of fatty acids.

According to a second variant, said antioxidant is a compound that is solid at 20°C, preferably a compound that has a melting point of between 100 and 140°C.

Particularly advantageously, said antioxidant is a phenolic antioxidant that does not contain a long alkyl chain of fatty acids and that has a melting point of between 100 and 140°C.

Most preferably, said antioxidant is tetrakis[methylene-(3,5-di-tert-butyl-4-hydroxyhydrocinnamate)]methane which has the following structure and which is hereinafter referred to as (AO-18).

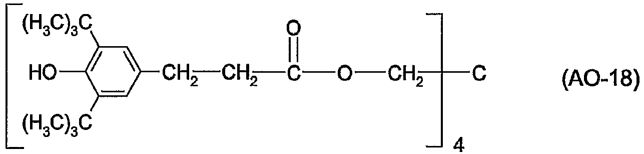

It has now been found that, under the specific conditions of the process that is the subject of the invention, the mixing of this compound with an aqueous alditol acetal composition such as, for example, an MDBS filter cake, can make it possible to obtain, after a conventional drying step, a pulverulent composition having simultaneously, with regard to a conventional MDBS powder:
a significantly increased packed bulk density,
a significantly increased particle size,
a significantly improved dispersibility in plastics,
a lack of adhesive capacity, and therefore improved flow properties.

This is without harming the organoleptic characteristics (in particular colour and odour characteristics) and applicative characteristics (in particular as a nucleating agent or a clarifying agent for plastics) of the resulting pulverulent composition.

This is unlike an additive such as calcium stearate which, under the same conditions, does not make it possible to simultaneously provide all these effects and/or to provide them to the same degree.

This is also unlike a composition consisting of a simple physical mixture of, firstly, a commercial MDBS powder and, secondly, a powder of the same antioxidant (AO-18).

In the context of the process that is the subject of the invention, it is preferred to mix the aqueous alditol acetal composition as defined above and the antioxidant or the antioxidant composition according to a weight ratio of between 50/1 and 4/1, expressed as dry weight of aqueous alditol acetal composition to dry weight of the antioxidant or antioxidant composition.

Even more advantageously, this weight ratio is between 25/1 and 5/1, in particular between 20/1 and 6/1.

As indicated, said antioxidant or antioxidant composition can be used in solid or liquid form.

By way of example, it is possible to mix a powder of the antioxidant such as the abovementioned antioxidant (AO-18), and the MDBS filter cake containing 50% SC and fragmented as described above, in a device of the "arch-breaking" type located immediately after the fragmentation device.

It is also possible to pulverize, disperse or spray premolten (AO-18) antioxidant on an MDBS cake that has already been neutralized but that is still in the course of being treated on the belt filter or the belt press and that has not yet undergone, in particular, the slightest fragmentation step.

The mixing step a) of the process according to the invention can last from a few seconds to several hours.

The drying step b) is then carried out with a view to converting the resulting mixture, a wet material that does not flow freely, into a dry and pulverulent composition.

This step b) can be carried out on any device, such as vacuum driers, fluidized bed driers, pneumatic driers, "flash" driers, microwave driers, etc.

According to a particular preferred embodiment, it is carried out at a relatively low temperature, i.e. at most equal to 150°C, preferably between 50 and 150°C, and in particular between 55°C and 145°C. The highest temperature reached by the mixture during said drying step b) may advantageously be between 90°C and 145°C, in particular between 110°C and 140°C.

As a result of this, a means is provided that makes it possible to obtain pulverulent alditol acetal compositions that have improved physical characteristics, and in particular:
that flow freely,
that have a packed bulk density, as will be defined elsewhere, at least equal to 0.30 kg/l, preferably between 0.35 and 0.55 kg/l, and/or
that have a particle size index "d 97", as will be defined elsewhere, of greater than 30 microns, in particular of between 35 and 100 microns.

This means makes it possible to prepare, in particular, as a new industrial product, a pulverulent alditol acetal composition, which contains from 2 to 20% by weight of at least one phenolic antioxidant, these percentages being expressed as total dry weight of antixidant(s) relative to the total dry weight of said composition.

The phenolic antioxidant can in particular be a compound that does not contain a long alkyl chain of fatty acids and/or that is solid at 20°C.

Preferably, said pulverulent composition flows freely.

Very advantageously, this novel composition is also characterized in that:
the alditol acetal is chosen from DBS and alkylated derivatives of DBS, and
the phenolic antioxidant is the above mentioned antioxidant (AO-18), i.e. is tetrakis[methylene-3-(3,5-di-(tert)-butyl-4-hydroxyhydrocin namate)] methane.

All the compositions described above are particularly suitable for preparing or stabilizing plastics or gelled materials or for preparing additives intended for said plastics or gelled materials.

It will be possible to understand the invention even more thoroughly from the examples that follow, which are not intended to limit the present invention and only given merely for the purpose of illustration.

### EXAMPLE 1:

An aqueous alditol diacetal composition that can be used according to the present invention is prepared from a dispersion, containing 5% SC, of fine particles of MDBS. This dispersion can be, for example, synthesized in an aqueous phase in accordance with the general teachings of US-A-5,023,354.

Said dispersion is filtered through an RT-type vacuum belt filter provided by the company Pannevis BV. In this same device, the filter cake is then washed by spraying hot water (approximately 80°C) and then neutralized by spraying a solution of water containing sodium, also hot, and, finally, pressed on a belt press, also functioning under vacuum and equipped with four pneumatically-driven pressing rollers.

The neutralized filter cake thus obtained has a solids content (SC) of approximately 50% and a temperature of approximately 45°C.

As it is unloaded from the device, it is fragmented by means of a set of 3 stainless steel wires 1.5 mm in diameter, stretched transversally to the direction of unloading.

The MDBS cake thus fragmented drops directly into a D2 17 DMR 100 device of the "arch-breaking/metering" type, equipped with a scraper arm and provided by the company Pari. It thus constitutes an aqueous alditol diacetal composition having a selected SC, that can be used in the process according to the invention.

This aqueous composition is mixed, in the same arch-breaking device, with the pulverulent commercially available antioxidant (AO-18) (= IRGANOX^{®} 1010 as sold by the company Ciba Specialty Chemicals, Inc.).

The mixing step a) is here carried out with a weight ratio, expressed on a dry basis, of firstly the MDBS filter cake to the antioxidant of approximately 6.7/1.

This step continues in a conveyor worm which then feeds a drying device for approximately 5 hours.

The drying step b) in accordance with the invention is here carried out in a vacuum drier with a total volume of 2360 litres, provided by the company Guedu and equipped with a jacket supplied with steam under a pressure of 2 bar (temperature: approximately 140°C). The drying is maintained until the temperature reached by the mixture is at least 130°C and in particular is of the order of 133 ± 2°C.

After approximately 14 hours of drying, a pulverulent composition of alditol acetal, in this instance of MDBS, containing approximately 13.4% (on a dry basis) of the antioxidant (AO-18), is in the present case obtained, which:
flows freely,
has no adhesive capacity,
has organoleptic characteristics, in particular whiteness and odour characteristics, that are at least equivalent to those of the commercial MDBS powders, exhibiting even improved characteristics in terms of miscibility and dispersibility in polymers of fluffy or pelletized form.

This novel MDBS composition (hereinafter denoted "COMPOSITION A") also has improved density and particle size characteristics, in particular packed bulk and "d97" particle size index characteristics.

The packed bulk density is studied conventionally, according to general principles such as those mentioned in the paragraph "<616> BULK DENSITY AND TAPPED DENSITY" of the USP pharmacopoeia.

In the present case, this packed bulk density or apparent volume mass of the packing, expressed in kg/l, is determined by means of a "STAV 2003"-type tapping volumeter provided by J. Engelsmann AG, according to the protocol below.

The cylinder (standardized 250 ml graduated measuring cylinder) containing the powder is subjected to a first series of 10 taps. The apparent density of the powder thus tapped is then determined.

The apparent density of the same powder introduced into the same cylinder but having undergone, respectively, 20, 30, 40, 50, 100, 150, 200, 250 and, finally 500 taps, is determined in the same way.

The tapped density of the powder in question corresponds to the highest value, in kg/l, thus determined, it having been possible for the "maximum" value to have been reached even before the cylinder had undergone the 500 taps and, for example, this value not having changed significantly between the test carried out with 100 taps and that carried out with 500 taps.

Thus, COMPOSITION A according to the invention, obtained in accordance with the present EXAMPLE 1, had a tapped density of 0.45 kg/l.

The "d97" particle size index of a powder is a value, expressed in microns (µm), that has been conventionally used for several decades to illustrate the maximum particulate size of a powder.

More precisely, if a powder is here characterized by a "d97" of X µm, this means that 97%, by volume, of the particles constituting said powder have a particulate size less than these X µm, said size here being determined:
by liquid-phase laser diffraction particle sizing, and
according to the method described below.

According to this method, 50 mg of the pulverulent composition to be studied are dispersed, with stirring for 2 minutes (magnetic stirrer at 600 rpm), in 20 ml of water from which the bubbles have been removed beforehand (degassing with helium) and containing 0.2%, by weight, of a nonionic surfactant of the ethoxylated alkyl phenol type, in this instance of Nonarox^{®}1030 provided by the company Seppic SA. The resulting dispersion is subjected, at ambient temperature, to ultrasound treatment for 15 minutes and at a power of 50 W.

The particle size characteristics of said dispersion thus treated are determined on a laser particle sizer, in this instance of the "Coulter LS 230" type from Beckman Coulter, equipped with a small volume module and using degassed water at ambient temperature containing 0.08%, by weight, of Nonarox^{®}1030 as carrier medium.

A "blank" is realized with respect to this carrier medium in accordance with the recommendation of the laser particle sizer instruction manual.

The particle sizing measurement is carried out according to the Fraunhofer theory, very commonly used as a method of calculation, and by selecting the PIDS (Polarization Intensity

Differential Scattering) module. The pump rate is set at position 30. The duration of each analysis is 90 seconds. For each composition, 3 analyses are carried out.

According to the method, which has just been given in detail, a mean "d97" index value of 97.3 µm is obtained for COMPOSITION A in accordance with the invention.

By scanning electromicroscopy, said COMPOSITION A exhibits, alongside non-agglomerated primary MDBS particles, a very large number of agglomerates whose size is very variable and is generally between about ten µm and several hundred µm.

When a photograph of COMPOSITION A is taken on a scanning electromicroscope (Quanta FEG 200), at a 340-times magnification, the presence of many agglomerates more or less ovoid, or even virtually spherical, in shape, generally between approximately 30 and 120 µm in size and made up of a multitude of primary MDBS particles caught up with one another, is observed. Given the "d97" value found above, i.e. almost 100 µm, it may be concluded that these agglomerates are sufficiently solid to have not been significantly destroyed by the conditions, which are nevertheless drastic, of dispersion and of treatment used for the purpose of/during the particle sizing measurement.

Applicative tests showed, moreover, that COMPOSITION A could perfectly well be used as a nucleating agent, and in particular a clarifying agent, for polyolefins.

In particular, under identical conditions, this COMPOSITION A does not generate significantly more "white spots" in a composition of polypropylene than a commercial MDBS powder.

### EXAMPLE 2:

As described above for COMPOSITION A, the tapped density and "d97" particle size index characteristics of COMPOSITIONS B to D in accordance with the invention and T1 and T2 as comparisons are determined.

These compositions are obtained, respectively, in the following way:
COMPOSITION B obtainable under the same conditions as COMPOSITION A, from an MDBS filter cake containing a 50% SC, except that the conditions for drying the mixture are modified such that said mixture reaches a temperature of the order of 133 ± 2°C in approximately 16 hours (instead of approximately 14 hours).
COMPOSITION C obtainable under the same conditions as COMPOSITION A, except that a) the weight ratio (on a dry basis) of the MDBS filter cake (containing a 50% SC) to IRGANOX^{®}1010 is 8/1 (instead of 6.7/1), and b) the drying lasts approximately 17 hours 30 minutes.
COMPOSITION D obtainable under the same conditions as COMPOSITION A, except that a) the weight ratio (on a dry basis) of the MDBS filter cake (containing a 50% SC) to IRGANOX^{®}1010 is 10/1 (instead of 6.7/1), and b) the drying lasts approximately 13 hours 10 minutes.
COMPOSITION T1 obtainable by simple physical mixing, carried out for 5 hours and at 30°C, of a commercial MDBS powder having a tapped density of less than 0.3 kg/l and a "d97" index of less than 30 µm and the IRGANOX^{®}1010 antioxidant, the weight ratio (on a dry basis) of the MDBS powder to the antioxidant powder being 10/1.
COMPOSITION T2 obtainable under the same conditions as COMPOSITION A, except that the additive mixed with the MDBS filter cake according to a weight ratio (on a dry basis) of 6.7/1 does not consist of a phenolic antioxidant but of calcium stearate.

The table below reiterates the mean values for tapped density ("TD" in kg/l) and for "d97" particle size index ("d97" in µm) of COMPOSITIONS A to D, T1 and T2.

| | TD | d97 |
|---|---|---|
| | kg/l | µm |
| COMPOSITION A | 0.45 | 93.5 |
| COMPOSITION B | 0.47 | 62.3 |
| COMPOSITION C | 0.47 | 58.4 |
| COMPOSITION D | 0.48 | 67.4 |
| COMPOSITION T1 | 0.28 | 43.1 |
| COMPOSITION T2 | 0.30 | 24.3 |

These results confirm that, by carrying out the process according to the invention, it is possible to obtain pulverulent alditol acetal compositions having density and particle size characteristics that are simultaneously and significantly improved.

This improvement cannot be achieved for a simple physical mixture of a commercial alditol acetal powder and the antioxidant (COMPOSITION T1).

Substitution of the IRGANOX^{®}1010 antioxidant with calcium stearate (COMPOSITION T2) does not make it possible, either, to achieve such an improvement, in any case under the conditions envisaged here.

This substitution also reduces the free-flowing capacity of COMPOSITION T2, which has a certain adhesive capacity linked to the very nature of the calcium stearate.

## Claims

1. A process for preparing a pulverulent alditol acetal composition, which comprises
a) mixing, in the presence or absence of other constituents, an aqueous alditol acetal composition having a solids content (SC) of between 40 and 75%, relative to the weight of the total aqueous alditol acetal composition,
and
an antioxidant in solid or liquid form or an antioxidant composition in solid or liquid form, containing one or more antioxidants, and
b) drying of the mixture obtained under a).

2. A process according to claim 1, wherein the alditol acetal is 1,3:2,4-di(benzylidene) sorbitol ("DBS") or a derivative thereof or 1,3:2,4-di(benzylidene) xylitol ("DBX") or a derivative thereof.

3. A process according to claim 1, wherein the alditol acetal is
1,3:2,4-di-(4-ethylbenzylidene) sorbitol,
1,3:2,4-di-(4-methylbenzylidene) sorbitol,
1,3:2,4-di-(3-methylbenzylidene) sorbitol, or
1,3:2,4-di-(3,4-dimethylbenzylidene) sorbitol.

4. A process according to claim 1, wherein the aqueous alditol acetal composition is a filter cake obtained after synthesis, in an aqueous medium, of the alditol acetal.

5. A process according to claim 1, wherein the antioxidant is a phenol derivative.

6. A process according to claim 1, wherein the antioxidant is a compound that is solid at 20°C.

7. A process according to claim 1, wherein the antioxidant is a phenol derivative which does not contain a long alkyl chain of fatty acids and which has a melting point of between 100 and 140°C.

8. A process according to claim 1, wherein the antioxidant is tetrakis[methylene-(3,5-di-tert-butyl-4-hydroxyhydrocin namate)] methane.

9. A process according to claim 1, wherein
in step a), the aqueous alditol acetal composition and the antioxidant or antioxidant composition are mixed in a weight ratio of between 50/1 and 4/1, expressed as dry weight of the aqueous alditol acetal composition to dry weight of the antioxidant or the antioxidant composition.

10. A process according to claim 9, wherein said weight ratio is between 25/1 and 5/1.

11. A process according to claim 1, wherein
step b) is carried out at a temperature at most equal to 150°C.

12. A process according to claim 11, wherein
in the drying step b), the highest temperature reached by the mixture is between 90°C and 145°C.

13. A pulverulent alditol acetal composition obtainable according to claim 1, which has a tapped density of at least equal to 0.30 kg/l.

14. A pulverulent alditol acetal composition obtainable according to claim 1, which has a "d97" particle size index of greater than 30 microns.

15. A pulverulent alditol acetal composition, which
contains from 2 to 20% by weight of one or more antioxidants, these percentages being expressed as total dry weight of the antioxidant(s) relative to the total dry weight of said composition,
has a tapped density of between 0.35 and 0.55 kg/l, and
has a "d97" particle size index of between 35 and 100 microns.

16. A composition according to claim 15, wherein the antioxidant is a phenol derivative which does not contain a long alkyl chain of fatty acids and/or which is solid at 20°C.

17. A composition according to claim 15, wherein
the alditol acetal is 1,3:2,4-di(benzylidene) sorbitol or an alkylated derivative of 1,3:2,4-di(benzylidene) sorbitol, and
the phenolic antioxidant is tetrakis[methylene-(3,5-di-tert-butyl-4-hydroxyhydrocinnamate)] methane.

18. The use of a pulverulent alditol acetal composition obtainable according to claim 1 for preparing plastics or gelled materials or for preparing additives intended for said plastics or gelled materials.

19. A process according to claim 1, wherein the aqueous alditol acetal composition has a solids content (SC) of between 47 and 72%, relative to the weight of the total aqueous alditol acetal composition.

## Patentansprüche

1. Verfahren zur Herstellung einer pulverförmigen Alditolacetalzusammensetzung, bei dem man
a) in Gegenwart oder Abwesenheit anderer Bestandteile eine wässrige Alditolacetalzusammensetzung mit einem Feststoffgehalt (FG) zwischen 40 und 75%, bezogen auf das Gewicht der gesamten wässrigen Alditolacetalzusammensetzung,
und
ein Antioxidans in fester oder flüssiger Form oder eine Antioxidanszusammensetzung in fester oder flüssiger Form, die ein oder mehrere Antioxidantien enthält, mischt und
b) die unter a) erhaltene Mischung trocknet.

2. Verfahren nach Anspruch 1, bei dem es sich bei dem Alditolacetal um 1,3:2,4-Di(benzyliden)sorbitol ("DBS") oder ein Derivat davon oder 1,3:2,4-Di(benzyliden)xylitol ("DBX") oder ein Derivat davon handelt.

3. Verfahren nach Anspruch 1, bei dem es sich bei dem Alditolacetal um
1,3:2,4-Di(4-ethylbenzyliden)sorbitol,
1,3:2,4-Di(4-methylbenzyliden)sorbitol,
1,3:2,4-Di(3-methylbenzyliden)sorbitol oder
1,3:2,4-Di(3,4-dimethylbenzyliden)sorbitol handelt.

4. Verfahren nach Anspruch 1, bei dem es sich bei der wässrigen Alditolacetalzusammensetzung um einen nach der Synthese des Alditolacetals in wässrigem Medium erhaltenen Filterkuchen handelt.

5. Verfahren nach Anspruch 1, bei dem es sich bei dem Antioxidans um ein Phenolderivat handelt.

6. Verfahren nach Anspruch 1, bei dem sich bei dem Antioxidans um eine Verbindung handelt, die bei 20°C fest ist.

7. Verfahren nach Anspruch 1, bei dem es sich bei dem Antioxidans um ein Phenolderivat handelt, das keine lange Alkylkette von Fettsäuren enthält und einen Schmelzpunkt zwischen 100 und 140°C aufweist.

8. Verfahren nach Anspruch 1, bei dem es sich bei dem Antioxidans um Tetrakis[methylen(3,5-di-tert-butyl-4-hydroxyhydrocinnamat)]methan handelt.

9. Verfahren nach Anspruch 1, bei dem man in Schritt a) die wässrige Alditolacetalzusammensetzung und das Antioxidans bzw. die Antioxidanszusammensetzung in einem Gewichtsverhältnis zwischen 50/1 und 4/1, ausgedrückt als Trockengewicht der wässrigen Alditolacetalzusammensetzung zu Trockengewicht des Antioxidans bzw. der Antioxidanszusammensetzung, mischt.

10. Verfahren nach Anspruch 9, bei dem das Gewichtsverhältnis zwischen 25/1 und 5/1 liegt.

11. Verfahren nach Anspruch 1, bei dem Schritt b) bei einer Temperatur von höchstens 150°C durchgeführt wird.

12. Verfahren nach Anspruch 11, bei dem im Trocknungsschritt b) die höchste Temperatur, die von der Mischung erreicht wird, zwischen 90°C und 145°C liegt.

13. Pulverförmige Alditolacetalzusammensetzung, die nach Anspruch 1 erhältlich ist und eine Stampfdichte von mindestens 0,30 kg/l aufweist.

14. Pulverförmige Alditolacetalzusammensetzung, die nach Anspruch 1 erhältlich ist und einen "d97"-Teilchengrößenindex von mehr als 30 Mikron aufweist.

15. Pulverförmige Alditolacetalzusammensetzung, die 2 bis 20 Gew.-% eines oder mehrerer Antioxidantien enthält, wobei diese Gewichtsangaben als gesamtes Trockengewicht des Antioxidans bzw. der Antioxidantien, bezogen auf das gesamte Trockengewicht der Zusammensetzung, ausgedrückt sind,
eine Stampfdichte zwischen 0,35 und 0,55 kg/l aufweist und
einen "d97"-Teilchengrößenindex zwischen 35 und 100 Mikron aufweist.

16. Zusammensetzung nach Anspruch 15, wobei es sich bei dem Antioxidans um ein Phenolderivat handelt, das keine lange Alkylkette von Fettsäuren enthält und/oder bei 20°C fest ist.

17. Zusammensetzung nach Anspruch 15, wobei
es sich bei dem Alditolacetal um 1,3:2,4-Di(benzyliden)sorbitol oder ein alkyliertes Derivat von 1,3:2,4-Di(benzyliden)sorbitol handelt und
es sich bei dem phenolischen Antioxidans um Tetrakis[methylen(3,5-di-tert-butyl-4-hydroxyhydrocinnamat)]methan handelt.

18. Verwendung einer nach Anspruch 1 erhältlichen pulverförmigen Alditolacetalzusammensetzung zur Herstellung von Kunststoffen oder gelierten Materialien oder zur Herstellung von Additiven für die Kunststoffe oder gelierten Materialien.

19. Verfahren nach Anspruch 1, bei dem die wässrige Alditolacetalzusammensetzung einen Feststoffgehalt (FG) zwischen 47 und 72%, bezogen auf das Gewicht der gesamten wässrigen Alditolacetalzusammensetzung, aufweist.

## Revendications

1. Procédé de préparation d'une composition pulvérulente d'acétal d'alditol, **caractérisé en ce qu'**il comprend :
a) une étape de mélange, en présence ou non de tiers constituants, d'une composition aqueuse d'acétal d'alditol présentant une teneur en matière sèche (MS) comprise entre 40 et 75 %, par rapport au poids de l'intégralité de la composition aqueuse d'acétal d'alditol, et
d'un agent antioxydant se présentant sous forme solide ou liquide ou d'une composition d'agent antioxydant se présentant sous forme solide ou liquide, comprenant un ou plusieurs agents antioxydants, et
b) une étape de séchage du mélange obtenu à l'étape a).

2. Procédé selon la revendication 1, **caractérisé en ce que** l'acétal d'alditol est le 1,3-2,4-di(benzylidène) sorbitol (« DBS ») ou un dérivé de celui-ci, ou le 1,3-2,4-di(benzylidène) xylitol (« DBX ») ou un dérivé de celui-ci.

3. Procédé selon la revendication 1, **caractérisé en ce que** l'acétal d'alditol est
le 1,3-2,4-di-(4-éthylbenzylidène) sorbitol,
le 1,3-2,4-di-(4-méthylbenzylidène) sorbitol,
le 1,3-2,4-di-(3-méthylbenzylidène) sorbitol, ou
le 1,3-2,4-di-(3,4-diméthylbenzylidène) sorbitol.

4. Procédé selon la revendication 1, **caractérisé en ce que** la composition aqueuse d'acétal d'alditol consiste en un gâteau de filtration obtenu après synthèse, en milieu aqueux, de l'acétal d'alditol.

5. Procédé selon la revendication 1, **caractérisé en ce que** l'agent antioxydant est un dérivé phénolique.

6. Procédé selon la revendication 1, **caractérisé en ce que** l'agent antioxydant est un composé qui est solide à 20 °C.

7. Procédé selon la revendication 1, **caractérisé en ce que** l'agent antioxydant est un dérivé phénolique qui ne contient pas de longue chaîne alkyle d'acides gras et qui présente un point de fusion compris entre 100 et 140 °C.

8. Procédé selon la revendication 1, **caractérisé en ce que** ledit agent antioxydant consiste en tétrakis-[méthylène-(3,5-di-tert-butyl-4-hydroxyhydrocinnamate)] méthane.

9. Procédé selon la revendication 1, **caractérisé en ce que** lors de l'étape a), on mélange la composition aqueuse d'acétal d'alditol et l'agent antioxydant ou la composition d'agent antioxydant selon un ratio pondéral compris entre 50/1 et 4/1, exprimé en poids sec de composition aqueuse d'acétal d'alditol sur le poids sec de l'agent antioxydant ou de la composition d'agent antioxydant.

10. Procédé selon la revendication 9, **caractérisé en ce que** ledit ratio pondéral est compris entre 25/1 et 5/1.

11. Procédé selon la revendication 1, **caractérisé en ce que** l'étape b) est menée à une température au plus égale à 150°C.

12. Procédé selon la revendication 11, **caractérisé en ce que**, lors de l'étape b) de séchage, la température la plus élevée atteinte par le mélange se situe entre 90°C et 145°C.

13. Composition pulvérulente d'acétal d'alditol pouvant être obtenue selon la revendication 1, **caractérisée en ce qu'**elle présente une densité tassée au moins égale à 0,30 kg/l.

14. Composition pulvérulente d'acétal d'alditol pouvant être obtenue selon la revendication 1, **caractérisée en ce qu'**elle présente un indice granulométrique « d 97 » supérieur à 30 microns.

15. Composition pulvérulente d'acétal d'alditol, **caractérisée en ce que** :
- elle contient de 2 à 20 % en poids d'au moins un agent antioxydant, ces pourcentages étant exprimés en poids sec total d'agent(s) antioxydant(s) par rapport au poids sec total de ladite composition,
- elle présente une densité tassée comprise entre 0,35 et 0,55 kg/l, et
- elle présente un indice granulométrique « d 97 » compris entre 35 et 100 microns.

16. Composition selon la revendication 15, **caractérisée en ce que** l'agent antioxydant est un dérivé phénolique qui ne contient pas de longue chaîne alkyle d'acides gras et/ou qui est solide à 20°C.

17. Composition selon la revendication 15, **caractérisée en ce que** :
- l'acétal d'alditol est le 1,3-2,4-di(benzylidène) sorbitol ou un dérivé alkylé de 1,3-2,4-di(benzylidène) sorbitol, et
- l'agent antioxydant phénolique est le tétrakis[méthylène-(3,5-di-tert-butyl-4-hydroxyhydrocinnamate)] méthane.

18. Utilisation d'une composition pulvérulente d'acétal d'alditol pouvant être obtenue selon la revendications 1 pour la préparation de matières plastiques ou gélifiées ou pour la préparation d'additifs destinés auxdites matières plastiques ou gélifiées.

19. Procédé selon la revendication 1, **caractérisé en ce que** la composition aqueuse d'acétal d'alditol présente une teneur en matière sèche (MS) comprise entre 47 et 72 %, par rapport au poids de l'intégralité de la composition aqueuse d'acétal d'alditol.
